Europäisches Patentamt

European Patent Office

Office européen des brevets

(11) Publication number: **0 092 302**
**B1**

# (12) EUROPEAN PATENT SPECIFICATION

(45) Date of publication of patent specification: 07.01.88

(51) Int. Cl.⁴: **C 12 N 5/00** // C12M3/00

(21) Application number: **83300860.0**

(22) Date of filing: **18.02.83**

(54) **Chemically specific surfaces for influencing cell activity during culture.**

(30) Priority: **16.04.82 US 369282**

(43) Date of publication of application:
**26.10.83 Bulletin 83/43**

(45) Publication of the grant of the patent:
**07.01.88 Bulletin 88/01**

(84) Designated Contracting States:
**BE DE FR GB IT NL SE**

(56) References cited:
**EP-A-0 073 562**

**CHEMICAL ABSTRACTS, vol. 86, 1977, page 365, no. 186888z, Columbus, Ohio, USA; L. SMITH et al.: "Glow discharge surface treatment for improved cellular adhesion" & POLYM. PREPR., AM. CHEM. SOC., DIV. POLYM. CHEM. 1975, 16(2), 186-190**

(73) Proprietor: **Becton, Dickinson and Company Mack Centre Drive P.O. Box 2224 Paramus New Jersey 07652-1149 (US)**

(72) Inventor: **Dunn, Terry S.**
**1300 Canterbury Road**
**Raleigh, N.C. (US)**
Inventor: **Williams, Joel L.**
**1306 Walnut Street**
**Cary, N.C. (US)**

(74) Representative: **Ruffles, Graham Keith et al MARKS & CLERK 57-60 Lincoln's Inn Fields London WC2A 3LS (GB)**

(56) References cited:
**CHEMICAL ABSTRACTS, vol. 81, 1974, page 4, no. 64028v, Columbus, Ohio, USA; T. MATSUDA et al.: "Modification and characterization of polystyrene surfaces used for cell culture" & J. POLYM. SCI., POLYM. CHEM. ED. 1974, 12(3), 489-496**

Courier Press, Leamington Spa, England.

## Description

The present invention relates generally to methods and articles for influencing cell growth, particularly tissue culture growth. More particularly, the invention relates to the modification of the surfaces of organic and inorganic substrates so as to provide a chemically modified surface which enhances or inhibits the growth of various cells in culture.

It is demonstrated herein that cellular function on polymer surfaces can be selectively controlled by varying the surface chemistry in contact with the cell membrane.

It is known that one of the factors influencing the growth of both primary cell cultures and established cell line cultures is the interface between the growth media in which the cultures are being grown and the substrate providing the boundary layer for the growth media. It is well known to modify the dispersed phase, i.e., the medium, in which a cell culture is being grown. It is also known to alter the surface interface between the medium and the substrate generally by coating the surface with various chemicals, such as collagen, for example. It is common laboratory practice to apply various chemical coatings to the substrate prior to attempting cell culture growth. Some chemicals which have been used include polylysine, collagen, hyaluronic acid, chondroitin 4-sulfate, chondroitin 6-sulfate, and various polysaccharides and lectins. The most commonly used chemical materials for modifying the substrate surface for primary cell culture growth are collagen, gelatin, and polylysine.

It is also known to use only tissue culture grade plastic for growing established adherent cell cultures. For example, commercially available tissue culture products are marketed under the trademark FALCON from Becton, Dickinson and Company. Although commercial tissue culture products appear to work well for established cell lines, they do not work well for primary cell types or certain cell types requiring collagen coating, for example.

In the present invention, the surface chemistry of a substrate is modified by subjecting the substrate to a plasma of a suitable material. It is known to subject substrates to plasmas of various types for accomplishing various purposes. For example, a brochure of International Plasma Corporation, Product Bulletin 24000, suggests that polystyrene tissue culture trays can be made wettable and receptive to living cells through plasma treatment with an oxygen or argon plasma. A brochure of Tegal Corporation, CP1287, indicates that plasmas can be used for dry stripping, cleaning and etching of electronic parts, cleaning optical surfaces, preparing surfaces having bonding, wettability and chemical resistance properties and dry cleaning of substrates to improve film deposition and adherence. The doctoral thesis of Lee M. Smith, "Cell Adhesion As Influenced By Substrate Surface Properties", Department of Material Science and Engineering, the University of Utah, 1978, p. 67, suggests that cell adherence is a function of the carbon/oxygen ratio of the surface. European Patent Specification 76562 published on 13 April 1983 discloses that the surfaces of organic and inorganic substrates are irreversibly modified by grafting specific chemical functional species onto the surface of the substrate by contacting the surface with selected components of a plasma of a vaporized material. The modified surfaces haved specific chemical functional groups grafted onto the surface and provide suitable surface chemistry for applications such as tissue culture products and protein-antibody binding or coupling.

None of these references, however, teach or suggest that cell growth can be influenced by chemical modification of the surface of the substrate on which the cell is grown.

It would be desirable to provide chemically specific substrate surfaces for the growth of selected cell cultures without the need for coating the substrate with chemical materials suitable for enhancement of the growth of primary cell types for example.

Due to the high growth rate generally exhibited by fibroblasts in culture, it is sometimes difficult to grow primary cells in culture because of the more rapid contaminating growth of fibroblasts. It would be desirable to provide specific substrate surfaces which are suitable for growth of cell cultures while inhibiting the growth of fibroblasts.

Accordingly, it is a principal object of the present invention to provide chemically specific surfaces for influencing the growth of various cell cultures at the interface between the growth media and the substrate.

It is another object of the present invention to provide a substrate with a chemically modified surface which influences growth of cell cultures on the surface.

It is a further object of the present invention to provide a substrate with a chemically modified surface which does not require coating with chemical compounds prior to cell culture growth on the substrate surface.

It is a still further object of the present invention to provide a method and an article for influencing the growth of cell cultures.

In accordance with the present invention, there is provided a method for growing a cell culture in a growth media which is in contact with a substrate surface, characterized in that the surface chemistry of the substrate is modified before use for cell culture growth by subjecting the surface of the substrate to a plasma which is produced from a plasma material which includes carbon dioxide sulphur dioxide or ammonia/carbon dioxide mixtures.

In general, it has been discovered that cell culture activity on the surface of a substrate can be controlled by varying the surface chemistry of a substrate surface in contact with the cell membrane. In particular, plasma modification processes may be used to provide specific chemical functional groups,

2

elemental ratios and other surface properties. In accordance with the invention, polymeric surfaces were prepared by plasma modification techniques which yielded a range of surface chemistries and properties. The modified polymeric surfaces were subjected to cell culture tests which indicate that cellular response is highly dependent on surface chemistry.

A plasma of a suitable material which includes carbon dioxide, sulphur dioxide or ammonia/carbon dioxide mixtures, may be used to modify the surface layer of organic and inorganic substrates, including polymeric materials, glass and metals regardless of thickness and which is independent of substrate composition, so as to provide specific chemical functional groups on the surface of the substrate. In the case of glass and metals, it is desirable to include a carbon source, such as methane, for example, to provide a thin organic layer on the inorganic substrate. Such plasma process is suitable for providing a surface for any cellular growth application whether the desired goal is to enhance or retard cell growth. The surface of the substrate is irreversibly modified by grafting specific chemical functional groups onto the surface with a plasma of a suitable material or with selected components of a plasma of a suitable material. The plasma material includes carbon dioxide, sulphur dioxide or ammonia/carbon dioxide mixtures, and may further comprise oxygen, carbon, hydrogen, nitrogen, halogen, sulfur, phosphorus, mixtures thereof and compounds thereof. The surfaces which are modified in accordance with the present invention have specific chemical functional groups grafted onto the substrate and provide suitable surface chemistry for tissue culture growth or suppression of growth.

In an embodiment of the present invention there is provided a process for modifying the surface of a substrate, specifically the modification of organic polymeric materials, by treatment with specific chemical species comprising:

providing a substrate with a reacton zone;

providing a suitable material in the reaction zone which includes carbon dioxide, sulphur dioxide or ammonia/carbon dioxide mixtures and forming a plasma which includes neutral material, positive ions of the material, negative ions of the material, electrons and photons;

preventing at least one of the components of the plasma from contacting the surface of substrate in the reaction zone;

contacting the surface of the substrate with the remainder of the components of the plasma in the reaction zone, thereby

forming specific functional groups of the material on the surface of the substrate.

This embodiment of the present invention is based on the discovery that utilization of selected reactive species of a plasma in contact with a substrate can be employed to provide specific chemical functional groups on the surface of the substrate which act to influence cell culture growth.

Reference is made to the accompanying drawings wherein:

Fig. 1 is a schematic diagram of an apparatus which can be employed in the practice of the invention.

Fig. 2 is a perspective view of a sample holder illustrated in the apparatus of Fig. 1.

Fig. 3 through 7 are x-ray photoelectron spectroscopy, ESCA, scans of various substrates, some of which have been treated in accordance with the invention.

Fig. 8 is a plot of the growth characteristics of primary neutronal cells as a function of surface chemistry and time.

Fig. 9 is a plot of the growth characteristics of primary fibroblasts as a function of surface chemistry and time.

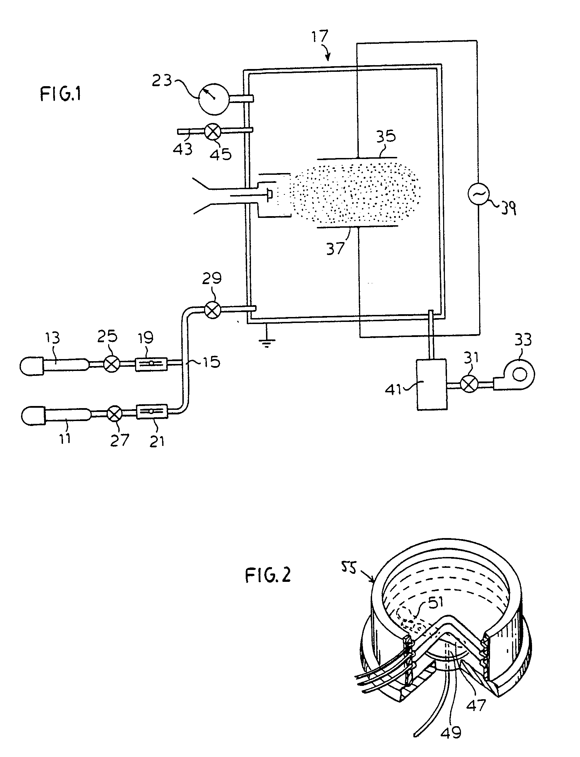

As shown in figure 1 there is provided a first gas reservoir 11, a second gas reservoir 13 with conduit means 15 to deliver either or both a first gas and a second gas to a vacuum chamber 17. Flow meters 19 and 21 are provided for measuring gas flow rate and a vacuum gauge 23 is provided to monitor the pressure within the vacuum chamber 17. Values 25, 27 and 29 are provided to regulate the flow rate of the gas in the first gas reservoir 11, the second gas reservoir 15 and the gas entering the vacuum chamber 17. Prior to use, the vacuum chamber 17 is evacuated by opening valve 31 to vacuum pump 33. Suitable electrodes 35 and 37 are connected to a suitable voltage source 39. The reactor system also includes a trap 41, vent conduit 43 and its valve 45.

As best seen in Fig. 2, a substrate 47 to be treated in accordance with the invention is placed in a sample holder 55 and disposed within the vacuum chamber 17. The sample holder includes a grid assembly of three grids, designated grid 1, grid 2 and grid 3. A collector 49 which can be electrically biased is disposed on the other side of the sample from the grid system. The sample is held in place beteen grid 3 and the collector by means of a suitable holder 51.

In operation, any or none of the three grids or the collector can be biased with positive or negative charge to repel selective components of a plasma generated between the electrodes 35 and 37. The substrate in the sample holder is then subjected to reaction with those components of the plasma which are not repelled.

The present invention can be employed to alter the surface of solid polymeric materials, including natural and synthetic addition and condensation polymers. Such polymeric materials include polyolefins, such as polyethylene, polypropylene, polyisobutylene and ethylene-alpha-olefin copolymers, acrylic polymers and copolymers, such as polyacrylate, polyethylethacrylate, polyethylacrylate; vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile, polyvinyl ketones; polyvinyl amindes; polyvinyl aromatics, such as polystyrene, polyvinyl esters such as polyvinyl acetate; copolymers of vinyl monomers with each other and olefins, such as ethyelen-methyl methacrylate copolymers, acrylonitrile styrene copolymers, ABS resins, and ethylenevinyl acetate copolymers; natural and synthetic rubbers, including butadiene-styrene copolymers, polyisoprene, synthetic polyisoprene, polybutadiene, butadiene-acrylonitrile copolymers, polychloroprene rubbers, polyisobutylene rubber, ethylene-propylene rubber, ethylene-propylene-diene rubbers, isobutylene-isoprene copolymers and polyurethane rubbers; polyamides, such as Nylon 66 and polycaprolactam; polyesters such as poly theylene terephthalate, alkyd resins; phenol-formaldehyde resins; ureaformaldehyde resins, malamine-formaldehyde resins; polycarbonates; polyoxymetyhylenes; polyimides; polyethers; epoxy resins, poly-urethanes; wool; cotton; silk; rayon; rayon-triacetate; cellulose; cellulose acetate; cellulose butyrate; cellulose acetatebutyrate; cellophane; cellulose nitrate; cellulose propionate; cellulose ethers; and carboxymethyl cellulose.

Inorganic materials, the surfaces of which can be modified in accordance with the invention, include non-metals such as graphite; metals, such as iron, aluminum, tin, copper and nickel; metal and other elemental oxides; such as magnesium oxide, silica, alumina and titania; minerals, such as clay, pyrite and asbestos; salts, such as sodium chloride and calcium carbonate; and such synthetic compositions such as glass and refractories.

The substrates whether organic or inorganic, can be any shape, such as continous or particulate, porous or impervious, and large or small. The invention can be employed for altering the surfaces of crystals, powders, plates, strips, films, sheets, wire, fibers, fabrics, filaments, tubing, and cast, extruded or compressed articles, and the like. Plasmas of carbon dioxide, sulphur dioxide and ammonia/carbon dioxide are employed in the present method. Other plasma materials can be included, and thus, more generally, plasmas of the elements of carbon, hydrogen, oxygen, nitrogen, sulfur, phosphorus, the halogens, mixtures thereof and compounds thereof are useful to modify the surface of substrates in accordance with the present invention.

Four types of plasmas can be generated; these are thermal plasmas, discharge plasmas, beam plasmas (such as ion beams and electron beams) and hybrid plasmas such as corona discharges. Suitable plasmas for use in the method of the present invention are discharge plasmas, beam plasmas and hybrid plasmas. Suitable plasmas can be generated by use of DC sources or AC sources having a frequency through the microwave range at power levels from about 1.0W to about 10 kW. Although not limited to high frequency sources, such as radiofrequency (r.f.) sources, plasmas of this type are particularly useful for the method and articles of the present invention in that selected plasma species concentrations can be controlled by source frequency, power, and system pressure. It is also true that r.f. plasmas are suitable for uniformly modifying the surface of more complex article shapes, such as tubing and other enclosures, for which DC and low frequency generated plasmas are not satisfactory.

In general, the r.f. plasmas suitable for use in the present invention are generated at a frequency of from about 1.0 to about 300 MHz at a power to initiate breakdown, such as from about 5 to about 1000 watts at pressures ranging from (say 0.133 Pa) 0.001 to 10 Torr (say 1330 Pa). The articles are usually subjected to the r.f. plasma for a period of from about 0.1 sec to about 120 minutes, although longer and shorter periods can be used. The plasma treatment can be followed by a quench cycle designed to provide an atmosphere suitable for reacting with residual active polymer species at or near the surface with pressures ranging from 1 Torr (say 133 Pa) to 760 Torr (say $1.01 \times 10^5$ Pa) for time periods of 1 second to 4 hours. The r.f. plasma can be generated between two flat plates, as shown in Figure 1, or can be generated with a helical coil, as shown in United States Patent No. 4,188,426.

In the following examples, a substrate of polystyrene is subjected to various plasma species. The substrate is then examined by x-ray photoelectron spectroscopy (ESCA) to determine the nature of the chemical functional groups on the surface of the polystyrene substrate.

After being subjected to the plasma of the following examples, the substrates are measured as to the Zeta Potential and Wilhelmy Plate Contact Angle. These values are listed in Table 1. These measurements of surface charge and surface tension characteristics illustrate the wide range of surface chemistries achieved. The culture growth plating efficiency is determined by inoculating petri dishes with a dilute cell suspension containing approximately 30 Human Diploid (MRC5) cells per milliliter in growth medium. The cell cultures were grown until the colonies ranged in size from 100 to 300 cells. The mean number of colonies for each test group of dishes was measured. The plating efficiency is defined as the ratio of the number of colonies per dish to the number of cells inoculated and is expressed as a percentage. The relative plating efficiency is determined by the ratio of the plating efficiency of the sample to that of the same sample grown on a petri dish identified by the Trademark FALCON obtained from Becton Dickinson and Company.

It is clear from Table 1 that the cellular response of fibroblasts is highly dependent on the surface chemistry produced by the plasma modification processes. The following examples show that fibroblast growth can be controlled by varying the surface chemistry in contact with the cell membrane.

The effect of specific surface chemistries on the growth of MRC5 fibroblasts as measured by relative plating efficiency was demonstrated with surfaces covering a range of carbon, oxygen, nitrogen and sulfur bonds.

4

## TABLE 1

| Plasma Precursor/Quench Gas | Zeta Potential zeta (mV) | Wilhelmy Plate $\theta_a$ (equil) | Relative Plating Efficiency MRC5 Fibroblasts |
|---|---|---|---|
| Falcon | −11.8 | 6±3 | 100 |
| $CO_2$ & $NH_3/NH_3$ | − 4.1 | 9±1 | 64.8 |
| $NH_3/CO_2$ | +31.3 | 64±3 | 31.3 |
| $SO_2/Air$ | −19.4 | 72±3 | 43 |
| $SO_2/SO_2$ | −14.8 | 77±1 | 0 |
| $CO_2/Air$ Untreated PS | − 9.7 | 101±1 | 6 |
| Glass | 0 | 48±3 | — |

### Example 1 $CO_2$/Air

Petri dishes were placed in the plasma chamber and evacuated to 75 μ over a 10-minute period and then filled with $CO_2$ to 180 μ pressure for two minutes. An r.f. plasma was produced for five minutes with a frequency of 11 MHz at 40 watts power. After treatment, the system was immediately opened to the atmosphere. The plating efficiency is listed in Table 1.

### Example 2 $CO_2$ and $NH_3/NH_3$

The petri dishes were placed in the plasma chamber and evacuated to 75 μ over a period of 10 minutes. $NH_3$ and $CO_2$ were bled into the system to 90 μ. An r.f. plasma at 11 MHz and 10 watts was produced for five minutes duration. The chamber was then brought up to 700 Torr (say $9.33 \times 10^4$ Pa) with $NH_3$ for a two-hour quench. The surface properties and plating efficiencies are listed in Table 1.

### Example 3 $NH_3/CO_2$

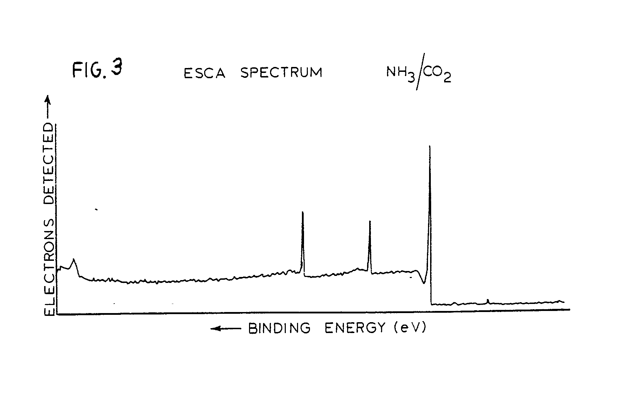

The petri dishes were placed in the plasma chamber and evacuated to 75 μ for 10 minutes and filled with 180 μ of $NH_3$ for two minutes. A 10 MHz 35 watt r.f. plasma was operated for five minutes. The system was then brought up to 700 Torr with $CO_2$ for a two-hour quench. Then the chamber was pumped down to 1 Torr and opened to atmosphere. Surface properties and plating efficiency are listed in Table 1. The ESCA data is given in Figure 3.

### Example 4 $SO_2$/Air

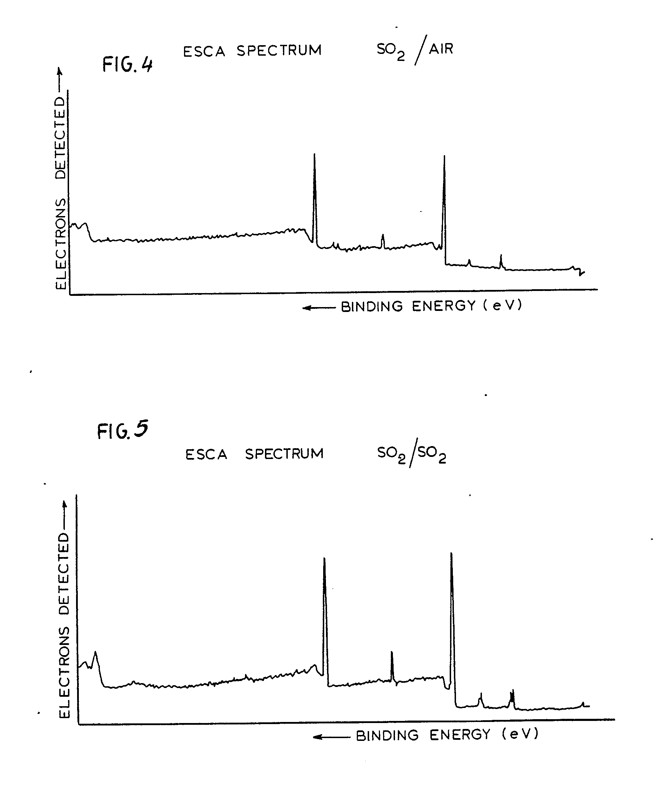

A plasma chamber containing polystyrene petri dishes was evacuated to 50 μ for 20 minutes, and then filled to 200 μ of $SO_2$ for 10 minutes. A 10MHz 34 watt r.f. plasma was operated for five minutes. The system was left at 200 μ for two minutes and then pumped down to 50 μ for two minutes, followed by opening to atmosphere. Surface properties and efficiency are listed in Table 1. The ESCA data is given in Figure 4. It is clear from this example that fibroblast growth is retarded by sulfur containing groups on the surface.

### Example 5 $SO_2/SO_2$

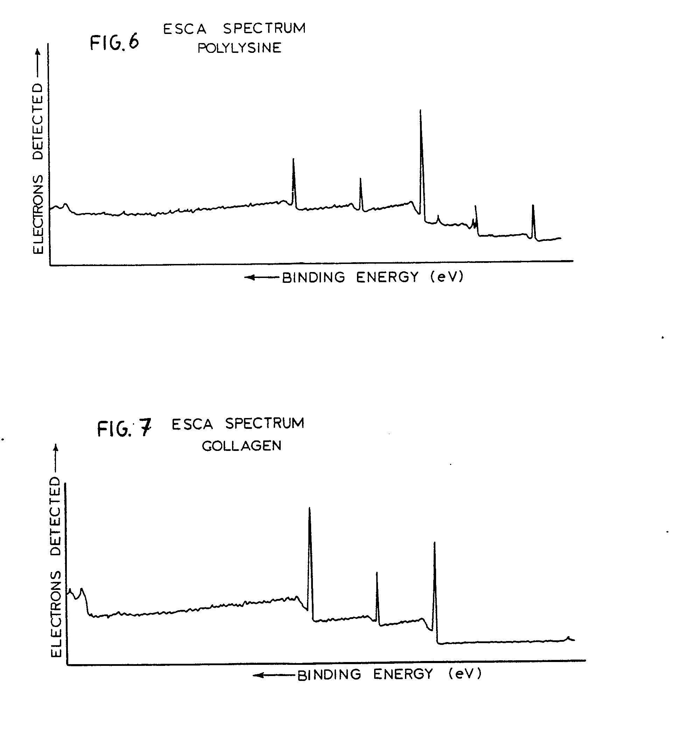

The petri dishes were placed into the plasma chamber and evacuated to 50μ for 20 minutes. $SO_2$ was inbled to 200 for 10 minutes. A 10 MHz 45 watt r.f. plasma was operated for five minutes. Then the system was immediately filled with $SO_2$ to a 700 Torr ($9.33 \times 10^4$ Pa) pressure for a two-hour quench. The system was pumped to 50μ and then opened to air. As illustrated in Table 1, the plating efficiency was negligible for fibroblasts on this surface. This provides a unique surface in that it slows or blocks fibroblast growth in culture. This is particularly useful in primary cultures where fibroblast growth can interfere with maintaining primary cells in culture. The surface chemistry as measured by ESCA is shown in Figure 5.

### Example 6

Polylysine and collagen coated petri dishes are used extensively in primary cell cultures. In this example, a plasma modification process was used to yield nitrogenous surfaces. This type of surface is described by $NH_3/CO_2$ as prepared in Example 3.

ESCA analysis of the $NH_3/CO_2$ surface yielded O/C = 0.14 and N/C = 0.19. The ESCA scan of this sample is shown in Figure 6.

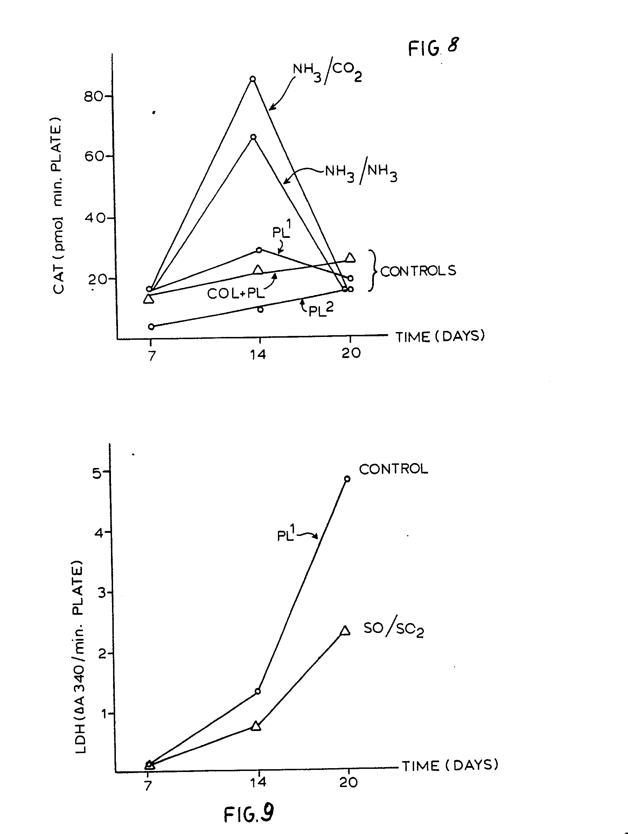

The response of primary neuronal cells in culture to the plasma treated surface with respect to polylysine and collagen/polylysine coated control surfaces demonstrates uniqueness in growth characteristics. Choline acetyltransferase, CAT, is a specific biochemical marker indicative of preferential neuronal cell support. As illustrated in Figure 7, the plasma modified nitrogenous surface supports synthesis of primary neurons to a much greater extent than any of the chemically coated surfaces.

Example 7

Plating efficiency test results with the MRC5 fibroblast cell line resulted in a significant reduction of growth on the two surfaces $SO_2$/Air and $SO_2$/$SO_2$ plasma treated as described in Examples 4 and 5 respectively.

The abundance of primary fibroblasts in neuronal cell cultures can be measured by LDH content. Lactate dehydrogenase, LDH, is a non-specific cell marker indicative of cytoplasma volume and, therefore, all cells present in culture including primary fibroblasts. As shown in Figure 8, this surface significantly slows down fibroblast growth compared to the control polylysine coated surface.

**Claims**

1. A method for growing a cell culture in a growth media which is in contact with a substrate surface, characterized in that the surface chemistry of the substrate is modified before use for cell culture growth by subjecting the surface of the substrate to a plasma which is produced from a plasma material which includes carbon dioxide, sulphur dioxide or ammonia/carbon dioxide mixtures.

2. A method in accordance with claim 1 wherein the plasma is a discharge plasma generated at a frequency of from 1 to 300 megahertz at a power of from 5 to 1000 watts at a pressure of from 0.133 to 1330 Pascals.

3. A method in accordance with claim 1 or 2 wherein the plasma material is ammonia/carbon dioxide mixtures.

4. A method in accordance with claim 1 or 2 wherein the plasma material is carbon dioxide.

5. A method in accordance with claim 1 or 2 wherein the plasma material is sulphur dioxide.

6. A method in accordance with any preceding claim, which comprises:

(a) positioning the substrate within a reaction zone;

(b) introducing a material into the reaction zone, the material including carbon dioxide, sulphur dioxide or ammonia/carbon dioxide mixtures;

(c) subjecting the material to plasma producing conditions, whereby the plasma comprises neutral material, positive ions of the material, negative ions of the material, electrons and photons;

(d) preventing at least one of the components of the plasma from contacting the surface of the substrate;

(e) contacting the surface of the substrate with the remainder of the components of the plasma, thereby

forming specific functional groups of the material on the surface of the substrate.

7. An article suitable for influencing the culture growth of a cell in contact therewith comprising a polymeric article whose surface chemistry has been modified by subjecting the surface to a plasma which is produced from a plasma material which is carbon dioxide, sulphur dioxide or an ammonia/carbon dioxide mixture.

8. An article suitable for influencing the culture growth of a cell in contact therewith comprising an inorganic article whose surface chemistry has been modified from a plasma material which is carbon dioxide, sulphur dioxide or an ammonia/carbon dioxide mixture.

**Patentansprüche**

1. Verfahren zum Wachsen einer Zellkultur in einem Wachstumsmedium, welches mit einer Substratoberfläche in Kontakt ist, dadurch gekennzeichnet, dass die Oberflächenchemie des Substrates vor der Verwendung zum Wachsen der Zellkultur modifiziert wurde, indem man die Oberfläche des Substrates einem Plasma aussetzte, das aus einem Plasmamaterial, welches Kohlendioxid, Schwefeldioxid oder Ammoniak/Kohlendioxid-Mischungen einschliesst, gebildet wurde.

2. Verfahren gemäss Anspruch 1, bei welchem das Plasma ein Entladungsplasma ist, das bei einer Frequenz von 1 bis 300 MHz und einer Leistung von 5 bis 1000 W bei einem Druck von 0,133 bis 1330 Pascale erzeugt wurde.

3. Verfahren gemäss Anspruch 1 oder 2, bei dem das Plasmamaterial eine Ammoniak/Kohlendioxid-Mischung ist.

4. Verfahren gemäss Anspruch 1 oder 2, bei dem das Plasmamaterial Kohlendioxid ist.

5. Verfahren gemäss Anspruch 1 oder 2, bei dem das Plasmamaterial Schwefeldioxid ist.

6. Verfahren gemäss einem der vorhergehenden Ansprüche, welches umfasst:

(a) Einbringen des Substrates in eine Reaktionszone,

(b) Einführen eines Materials in die Reaktionszone, wobei das Material Kohlendioxid, Schwefeldioxid oder Ammoniak/Kohlendioxid-Mischungen umfasst,

(c) Einwirkenlassen von plasmaproduzierenden Bedingungen auf das Material, wobei das Plasma neutrales Material, positive Ionen des Materials, negative Ionen des Materials, Elektronen und Photonen umfasst,

(d) Verhindern, dass wenigstens eine der Plasmakomponenten die Oberfläche des Substrates kontaktiert,

(e) Kontaktieren der Substratoberfläche mit dem Rest der Plasmakomponenten, wodurch spezifische funktionelle Gruppen des Materials an der Substratoberfläche ausgebildet werden.

7. Gegenstand zum Beeinflussen des Kulturwachstums einer damit in Kontakt befindlichen Zelle, umfassend einen polymeren Gegenstand, dessen Oberflächenchemie dadurch modifiziert wurde, dass man die Oberfläche einem Plasma aussetzt, welches von einem Plasmamaterial, das Kohlendioxid, Schwefeldioxid oder eine Ammoniak/Kohlendioxid-Mischung ist, gebildet wird.

8. Gegenstand zur Beeinflussung des Kulturwachstums einer damit in Kontakt befindlichen Zelle, umfassend einen anorganischen Gegenstand, dessen Oberflächenchemie mittels eines Plasmamaterials, welches Kohlendioxid, Schwefeldioxid oder eine Ammoniak/Kohlendioxid-Mischung ist, modifiziert wurde.

**Revendications**

1. Procédé pour fair croître une culture de cellules dans un milieu de croissance qui est en contact avec une surface de substrat, caractérisé en ce que la nature chimique de la surface du substrat est modifiée avant utilisation pour une croissance de culture de cellules en soumettant la surface du substrat à un plasma qui est produit à partir d'un matériau de plasma, qui contient du gaz carbonique, du dioxyde de soufre ou des mélanges d'ammoniac et de gaz carbonique.

2. Procédé selon la revendication 1, dans lequel le plasma est un plasma de décharge produit à une fréquence de 1 à 300 MHz à une puissance de 5 à 1000 watts et à une pression de 0,133 à 1330 Pa.

3. Procédé selon la revendication 1 ou 2, dans lequel le matériau de plasma est un mélange d'ammoniac et de gas carbonique.

4. Procédé selon la revendication 1 ou 2, dans lequel le matériau de plasma est du gaz carbonique.

5. Procédé selon la revendication 1 ou 2, dans lequel le matériau de plasma est du dioxyde de soufre.

6. Procédé selon l'une quelconque des revendications précédentes, qui consiste à:

(a) placer le sustrat à l'intérieur d'une zone de réaction;

(b) introduire dans la zone de réaction un matériau contenant du gas carbonique, du dioxyde de soufre ou des mélanges d'ammoniac et de gaz carbonique;

(c) soumettre le matériau à des conditions de production du plasma, d'où il résulte que le plasma comprend un matériau neutre, des ions positifs du matériau, des ions négatifs du matériau, des électrons et des photons;

(d) empêcher au moins l'un des composants du plasma de venir en contact avec la surface du substrat;

(e) mettre en contact la surface du substrat avec le reste des composants du plasma, de façon à former des groupes fonctionnels spécifiques du matériau sur la surface du substrat.

7. Produit convenant pour influencer la croissance de cultures d'une cellule en contact avec lui, comprenant un produit polymère dont la nature chimique de la surface a été modifiée en soumettant la surface à un plasma qui est produit à partir d'un matériau de plasma, qui est le gaz carbonique, le dioxyde de soufre ou un mélange d'ammoniac et de gaz carbonique.

8. Produit convenant pour influencer la croissance de cultures d'une cellule en contact avec lui, comprenant un produit inorganique dont la nature chimique de la surface a été modifiée à partir d'un matériau de plasma qui est le gaz carbonique, le dioxyde de soufre, ou un mélange d'ammoniac et de gaz carbonique.

0 092 302

FIG.1

FIG.2

1

FIG. 3    ESCA  SPECTRUM    $NH_3/CO_2$

ELECTRONS DETECTED →

← BINDING  ENERGY (eV)

0 092 302

2

FIG. 4    ESCA SPECTRUM    $SO_2$/AIR

ELECTRONS DETECTED →

← BINDING ENERGY ( eV )

FIG. 5

ESCA  SPECTRUM    $SO_2$/$SO_2$

ELECTRONS DETECTED →

← BINDING ENERGY ( eV )

FIG.6 ESCA SPECTRUM
POLYLYSINE

ELECTRONS DETECTED →

←BINDING ENERGY (eV)

FIG.7 ESCA SPECTRUM
GOLLAGEN

ELECTRONS DETECTED →

←BINDING ENERGY (eV)

FIG. 8

FIG. 9